# EUROPEAN PATENT SPECIFICATION

(11) **EP 1 748 791 B1**
(45) Date of publication and mention of the grant of the patent: **14.04.2010**
(21) Application number: 05745670.9
(22) Date of filing: 11.05.2005
(51) Int. Cl.: A61K 39/39, A61K 39/095, C07H 3/06, A61P 31/00

(54) **NEISSERIA MENINGITIDIS IgtB LOS AS ADJUVANT**
NEISSERIA MENINGITIDIS IgtB LOS ALS ADJUVANS
LOS DE NEISSERIA MENINGITIDIS IgtB UTILISES EN TANT QU'ADJUVANTS

(30) Priority: 11.05.2004 EP 04076401
(43) Date of publication of application: 07.02.2007
(73) Proprietor: De Staat der Nederlanden, vert. door de minister van VWS, 2500 EJ The Hague (NL)
(72) Inventor: STEEGHS, Liana, Juliana, Josephine, NL-3544 PP Utrecht (NL); VAN DE WINKEL, Johannes, Gerardus, Joseph, NL-3707 CK Zeist (NL); VAN DER LEY, Peter, André, NL-3572 DJ Utrecht (NL)
(74) Representative: Swinkels, Bart Willem
(86) International application number: PCT/NL2005/000358
(87) International publication number: WO 2005/107798

(56) References cited:
- WO-A-00/26384
- WO-A-2004/014417
- NL-A- 9 101 359
- LEY VAN DER P ET AL: "MODIFICATION OF LIPID A BIOSYNTHESIS IN NEISSERIA MENINGITIDIS IPXLMUTANTS: INFLUENCE ON LIPOPOLYSACCHARIDE STRUCTURE, TOXICITY AND ADJUVANT ACTIVITY" INFECTION AND IMMUNITY, AMERICAN SOCIETY OF MICROBIOLOGY, WASHINGTON, DC, US, vol. 69, no. 10, October 2001 (2001-10), pages 5981-5990, XP001055221 ISSN: 0019-9567
- STEEGHS LIANA ET AL: "Teasing apart structural determinants of 'toxicity' and 'adjuvanticity': implications for meningococcal vaccine development." JOURNAL OF ENDOTOXIN RESEARCH, vol. 10, no. 2, April 2004 (2004-04), pages 113-119, XP009038407 ISSN: 0968-0519

## Description

### Field of the invention

The present invention relates to Neisserial Lipo-Oligo-Saccharides (LOS) that have increased immunostimulatory activity. The Neisserial LOS's of the invention comprise a tri-saccharide outer core that shows increased binding to a receptor on dendritic cells. The tri-saccharide outer core of the Neisserial LOS's combined with a Lipid A moiety with reduced toxicity is useful as an adjuvant in vaccine preparations.

### Background of the invention

*Neisserial meningitidis* LPS has received attention as a potential vaccine candidate due to its capacity to induce LPS-specific immune responses as well as being a potent adjuvant. However, the endotoxic activity of LPS has limited its use in vaccines so far.

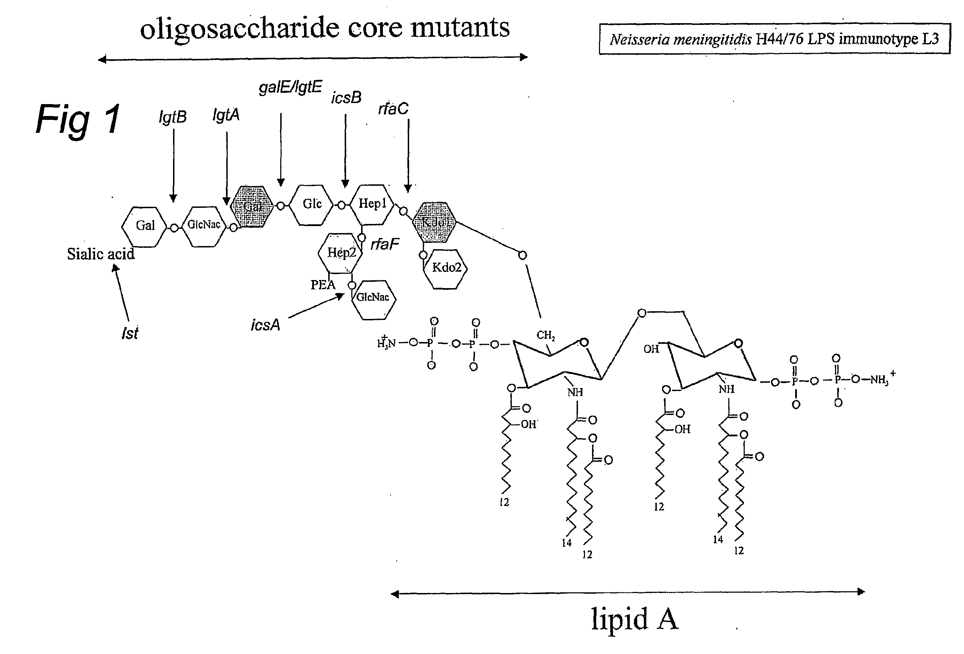

Meningococcal LPS is composed of a hydrophobic lipid A portion that anchors it to the bacterial outer membrane and a hydrophilic oligosaccharide core, which is exposed to the bacterial surface (Figure 1). Inclusion of the native oligosaccharide chain in vaccine preparations is unadvisable due to its structural similarity with host glycolipid antigens. Genetic engineering of the LPS oligosaccharide biosynthesis pathways has enabled the production of series of *N. meningitidis* mutants expressing truncated oligosaccharide chains (Figure 1). The endotoxic and adjuvant properties of LPS appear to be determined by the lipid A part of the molecule. We have previously generated a unique set of *N. meningitidis* lipid. A mutants with highly improved pharmacological properties. In particular, inactivation of the *lpxL1* (*htrB1*) gene involved in lipid A acyloxyacylation resulted in the isolation of a lipid A mutant expressing penta-acylated instead of hexa-acylated lipid A, with reduced endotoxic but retained adjuvant activity. In addition, a *N. meningitidis* mutant completely lacking LPS due to inactivation of the meningococcal *lpxA* gene was isolated. Thus, modification of the lipid A portion as well as the oligosaccharide portion of meningococcal LPS has opened new possibilities for development of meningococcal vaccines.

Dendritic cells (DC) have become of major interest because of their role in the initiation of an immune response both during natural infection and in response to vaccination. Immune responses to bacteria are initiated by DC, which phagocytose and process bacterial antigens for presentation to T cells. We have shown recently that *N. meningitidis* LPS plays a major role during interactions with human DC. Moreover LPS is required for internalisation of the bacteria and full activation of the DC.

WO2004/14417 discloses Neisserial bleb preparations derived from Neisserial lgtB⁻ strains. NL-A-9 101 359 discloses the use of the Neisseirial lgtB⁻ LPS in conjugation with a peptide. WO 00/26384, van der Ley et al. van der Ley et al., 2001, Infect.Immun. 69: 5981-5990, and Steeghs et al., 2004, J. Endotoxin Res. 10: 113-119 all disclose Neisserial LPS molecules with a modified lipid A moiety having a reduced toxicity but retained adjuvant activity.

### Description of the invention

### Definitions

Adjuvants are herein defined to include any substance or compound that, when used in combination with an antigen, to immunise a mammal, preferably a human, stimulates the immune system, thereby provoking, enhancing or facilitating the immune response against the antigen, preferably without generating a specific immune response to the adjuvant itself. Preferred adjuvants enhance the immune response against a given antigen by at least a factor of 1.5, 2, 2.5, 5, 10 or 20, as compared to the immune response generated against the antigen under the same conditions but in the absence of the adjuvant. Tests for determining the statistical average enhancement of the immune response against a given antigen as produced by an adjuvant in a group of animals or humans over a corresponding control group are available in the art. The adjuvant preferably is capable of enhancing the immune response against at least two different antigens. The adjuvant of the invention will usually be a compound that is foreign to a mammal, thereby excluding immunostimulatory compounds that are endogenous to mammals, such as e.g. interleukins, interferons and other hormones.

### Detailed description of the invention

The present invention is based on the surprising discovery that a Neisserial LPS with a particular tri-saccharide outer core structure, shows increased binding and internalisation by human dendritic cells (DC's). Binding and internalisation of the tri-saccharide-containing LPS, or rather tri-saccharide-containing LOS (lipooligosaccaride), is mediated by the C-type lectin DC-SIGN that is expressed on human DC's and results in activation and maturation of the DC's at increased rate. The improved immunostimulatory activity of this new Neisserial tri-saccharide-containing LOS allows its use as a potent adjuvant in immunisation and vaccination.

Thus, in a first aspect the invention relates to a method for immunisation or vaccination of a mammal with an antigen, the method comprising the administration (to the mammal) of the antigen and a Neisserial LOS is represented by the formula (I): 
KDO
α2→4
GlcNAc β1→3 Gal β1→4 Glc β1→4 Hep α1→5 KDO - LA
α1→3
Hep→PEA (3 or 6)
α1→2
GlcNAc,
 whereby LA is a wild-type lipid A moiety, preferably from a *Neisseria* strain, or more preferably a lipid A moiety with reduced toxicity.

In formula (1), GlcNAc defines D-glucosamine; Gal defines D-galactose; Glc defines D-glucose; Hep defines D-heptose; KDO defines 3-deoxy-D-manno-octulosonic acid; PEA defines phospho-ethanolamine; β1→4 defines a β-glycosidic linkage between the first and fourth positions; β1→3 defines a β-glycosidic linkage between the first and third positions; α1→5 defines a α-glycosidic linkage between the first and fifth positions; α1→3 defines a α-glycosidic linkage between the first and third positions; α1→2 defines a α-glycosidic linkage between the first and second positions; α2→4 defines a α-glycosidic linkage between the second and fourth positions; PEA(3 or 6) indicates that the phospho-ethanolamine may be linked at either the third or sixth position of the heptose or may be absent altogether.

The invention thus relates to the use of a Neisserial LOS as defined above in the manufacture of a medicament for immunisation or vaccination of a mammal. Preferably the Neisserial LOS of the invention is used as an adjuvant. More preferably, the Neisserial LOS is used in combination with an antigen in the manufacture of a medicament for raising an immune response against (or vaccination with) the antigen in the mammal.

In the methods and uses of the invention, the mammal preferably is a human. The antigen preferably is an antigen from or produced by a bacterium, a virus, a fungus, a parasite, a cancer cell or an allergen as further defined below. The antigen and Neisserial LOS are preferably used in the treatment and/or prevention of an infectious disease caused by the bacterium, virus, fungus, or parasite, or the tumor caused by the cancer cell, or the allergy caused by the allergen.

In preferred embodiments of the invention, the Neisserial LOS of the invention has a modified Lipid A moiety (LA) with reduced toxicity. The toxicity of the modified LA preferably is less than the toxicity of a corresponding wild-type LA, more preferably the modified LA is less than 90, 80, 60, 40, 20, 10, 5, 2, 1, 0.5 or 0.2% of the toxicity of the wild-type LA. The toxicities of wild-type and various modified LA's with reduced toxicity may be determined in any suitable assay known in the art. A preferred assay for determining the toxicity, i.e. the biological activity of the LA's or Neisserial LOS's of the invention is the WEHI test for TNF-alpha induction in the MM6 macrophage cell line (Espevik and Niessen, 1986, J.Immunol.Methods 95: 99-105; Ziegler-Heitbrock et al., 1988, Int.J.Cancer 41: 456-461).

On the other hand, the Neisserial LOS of the invention having an LA with reduced toxicity should still have sufficient immunostimulatory activity, i.e. adjuvant activity. The Neisserial LOS of the invention with reduced toxicity preferably has at least 10, 20, 40, 80, 90 or 100% of the immunostimulatory activity of the corresponding Neisserial LOS with a wild-type LA, whereby the immunostimulatory activity is determined by measuring the production of at least one cytokine or the expression of at least one costimulatory molecule as described in Example 3 herein.

Neisserial LOS's having reduced toxicity of the Lipid A moiety but retaining (part of) the adjuvant activity, may e.g. be obtained from genetically modified Gram negative pathogens and as reviewed in WO02/09746. In particular, preferred Neisserial LOS's with LA's with reduced toxicity include: (a) LOS's with a lipid A moiety as obtainable from a *Neisseria* strain with a mutation that causes a reduction or inactivation of the expression of one or more of the *lpxL1* and *lpxL2* genes (formerly known as *htrB* and *msbB*) genes or homologues thereof (see e.g. EP 1 127 137; US 5,997,881); (b) LOS's with a lipid A moiety as obtainable by removal of one or more secondary O-linked esterified fatty acids from a wild-type Lipid A moiety by treatment with an acyloxyhydrolase or alkaline hydrolysis (US 5,103,661; Erwin et al., 1991, Infect. Immun. 59: 1881-87); (c) LOS's with a lipid A moiety as obtainable by hydrazine treatment of a wild-type Lipid A moiety (Gu et al., 1996, Infect. Immun. 64: 4047-53; Polotsky et al., 1994, Infect. Immun. 62: 210-14; Gu et al., 1998, Infect. Immun. 66:1891-97, 1998); (d) LOS's with a lipid A moiety as obtainable by dephosphorylation of a Lipid A moiety with an alkaline phosphatase (US 6,290,952); and, (e) LOS's obtainable by enzymatic dephosphorylation or deacylation of LOS produced in Neisserial host in which a heterologous *lpxE* or *pagL* gene is expressed. Reduced expression is herein understood to mean increased or reduced when compared to a corresponding wild-type strain, whereby the expression level preferably at least differs by a factor 2, 5 or 10 from the corresponding wild-type strain.

Preferred LA's with reduced toxicity thus include LA with a secondary C₁₂-acyl only on the reducing glucosamine, LA with a secondary C₁₂-acyl only on the non-reducing glucosamine, LA lacking both secondary C₁₂-acyl groups, LA's lacking one or more phosphate groups from either the 1 or 4' position and combinations of the aforementioned deacylations and dephosphorylations.

In a further aspect the disclosure relates to a Neisserial LOS that is represented by the formula (I): 
KDO
α2→4
GlcNAc β1→3 Gal β1→4 Glc β1→4 Hep α1→5 KDO - LA
α1→3
Hep→PEA (3 or 6)
α1→2
GlcNAc,
 whereby preferably LA is a Lipid A moiety with reduced toxicity as defined above.

In yet another aspect the invention relates to a composition comprising a Neisserial LOS as herein defined above, a pharmaceutically acceptable carrier, and an antigen that is from or produced by a bacterium, a virus, a fungus, a parasite, a cancer cell or an allergen. The pharmaceutical compositions may further comprise pharmaceutically acceptable stabilizing agents, osmotic agents, buffering agents, dispersing agents, and the like. The preferred form of the pharmaceutical composition depends on the intended mode of administration and therapeutic application. The pharmaceutical carrier can be any compatible, non-toxic substance suitable to deliver the active ingredients, i.e. the Neisserial LOS and the antigen, to the patient. Pharmaceutically acceptable carriers for intranasal delivery are exemplified by water, buffered saline solutions, glycerin, polysorbate 20, cremophor EL, and an aqueous mixture of caprylic/capric glyceride, and may be buffered to provide a neutral pH environment. Pharmaceutically acceptable carriers for parenteral delivery are exemplified by sterile buffered 0.9% NaCl or 5% glucose optionally supplemented with a 20% albumin. Preparations for parental administration must be sterile. The parental route for administration of the active ingredients is in accord with known methods, e.g. injection or infusion by subcutaneous, intravenous, intraperitoneal, intramuscular, intraarterial or intralesional routes. The compositions of the invention are preferably administered by bolus injection. A typical pharmaceutical composition for intramuscular injection would be made up to contain, for example, 1 - 10 ml of phosphate buffered saline and 1 to 100 µg, preferably 15-45 µg of antigen and 1 to 100 µg, preferably 15-45 µg of the Neisserial LOS of the invention. For oral administration, the active ingredient can be administered in liquid dosage forms, such as elixirs, syrups, and suspensions. Liquid dosage forms for oral administration can contain coloring and flavoring to increase patient acceptance. Methods for preparing parenterally, orally or intranasally administrable compositions are well known in the art and described in more detail in various sources, including, for example, Remington's Pharmaceutical Science (15th ed., Mack Publishing, Easton, PA, 1980).

The antigen in the composition of the invention preferably is an antigen that is from or produced by a bacterium, a virus, a fungus, a parasite, a cancer cell or an allergen. Viral antigens that may be combined with the Neisserial LOS of the invention can be derived from all sorts of viruses, non-limiting examples of such viruses are: Retroviridae such as Human Immunodeficiency virus (HIV); a rubellavirus; paramyxoviridae such as parainfluenza viruses, measles, mumps, respiratory syncytial virus, human metapneumovirus; flaviviridae such as yellow fever virus, dengue virus, Hepatitis C Virus (HCV), Japanese Encephalitis Virus (JEV), tick-borne encephalitis, St. Louis encephalitis or West Nile virus; Herpesviridae such as Herpes Simplex virus, cytomegalovirus, Epstein-Barr virus; Bunyaviridae; Arenaviridae; Hantaviridae such as Hantaan; Coronaviridae; Papovaviridae such as human Papillomavirus; Rhabdoviridae such as rabies virus. Coronaviridae such as human coronavirus; Alphaviridae, Arteriviridae, filoviridae such as Ebolavirus, Arenaviridae, poxviridae such as smallpox virus, and African swine fever virus. Likewise the Neisserial LOS's of the invention may be combined with antigens derived from pathogenic bacteria, fungi (including yeasts), or parasites. Such antigens include bacterial antigens of e.g. *Helicobacter,* such as *H. pylori, Neisseria,* such as *N. mengitidis, Haemophilus,* such as *H. influenza, Bordetella,* such as *B. pertussis, Chlamydia, Streptococcus,* such as *Streptococcus* sp. serotype A, *Vibrio,* such as *V. cholera,* Gram-negative enteric pathogens including e.g. *Salmonella, Shigella, Campylobacter* and *Escherichia,* as well as antigen from bacteria causing anthrax, leprosy, tuberculosis, diphtheria, Lyme disease, syphilis, typhoid fever, and gonorrhea. Antigens from parasites e.g. include antigens from protozoans, such as *Babeosis bovis, Plasmodium, Leishmania* spp. *Toxoplasma gondii,* and *Trypanosoma,* such as *T. cruzi.* Fungal antigens may include antigens from fungi such as *Aspergillus* sp., *Candida albicans, Cryptococcus,* such as e.g *C. neoformans,* and *Histoplasma capsulatum.*

Although vaccination is generally applied for the prophylactic protection against pathogens or for the treatment of diseases following pathogenic infection, the person skilled in the art is aware of the application of vaccines for tumor-treatment Moreover, an increasing number of tumor-specific proteins are found to be proper entities that can be targeted by human or humanized antibodies. Such tumor-specific proteins are also within the scope of the present invention. Many tumor specific antigens are known in the art. Therefore, in one preferred embodiment, the invention provides compositions comprising a tumor-specific antigen and a Neisserial LOS as defined above. Suitable tumor antigens include e.g. carcinoembryonic antigen, prostate-specific membrane antigen, prostate specific antigen, protein MZ2-E, polymorphic epithelial mucin (PEM), folate-binding-protein LK26, (truncated) epidermal growth factor receptor (EGRF), HER2, Thomsen-Friedenreich (T) antigen, GM-2 and GD-2 gangliosides, Ep-CAM, mucin-1, epithialial glycoprotein-2, and colon specific antigen.

In addition, antigens can be targeted to DC's in order to induce tolerance in the prevention of auto-immune disease. Such allergens are also within the scope of the present invention.

In another aspect the disclosure relates to methods for producing a Neisserial LOS as defined above. A preferred method comprises the steps of: (a) culturing a *N. menigitidis* strain, whereby the strain expresses a Neisserial LOS of immunotype L2, L3 or L4 and lacks an active *lgtB* gene, or expresses a Neisserial LOS of immunotype L6, and whereby the strain carries a mutation that inactivates or reduces the expression of one or more of the *lpxL1* and *lpxL2* genes; and, (b) recovery of the Neisserial LOS. Alternatively, the method comprising the steps of: (a) culturing a *N. menigitidis* strain, whereby the strain expresses a Neisserial LOS of immunotype L2, L3 or L4 and lacks an active *lgtB* gene, or expresses a Neisserial LOS of immunotype L6; (b) recovery of the Neisserial LOS; and, (c) treating the Neisserial LOS with at least one of hydrazine, an alkaline phosphatase or acyloxyhydrolase to reduce the toxicity of the lipid A moiety. In the method, the order of steps (b) and (c) may be exchanged. In a preferred method, in step c) the toxicity is reduced until less than 80% of the toxicity of the wild-type Neisserial LOS, as determined in a WEHI assay as described above.

In yet another aspect the disclosure relates to Gram-negative OMV's (Outer Membrane Vessicles) or blebs comprising the Neisserial LOS's as defined above. Means and methods for producing Gram-negative OMV's or blebs are described in WO 02/09746. Gram-negative blebs thus obtained may be combined with the Neisserial LOS of the invention to produce Gram-negative blebs comprising the Neisserial LOS's. Alternatively the blebs may be produced from a Neisserial strain as defined above, that is capable of producing a Neisserial LOS of the invention. The Gram-negative blebs comprising may further be combined with an antigen as described above and/or with pharmaceutically acceptable carriers as described above.

### Description of the figures

**Figure 1****:** Schematic representation of *N. meningitidis* WT L3 LPS and its oligosaccharide mutants that can be generated by insertional inactivation of the genes (indicated in italic) encoding the glycosyltransferases.
**Figure 2****:** Dendritic cells (DC) were cultured with FITC-labelled bacteria at a ratio of 1:50 for 24h. At the indicated time points samples were taking to measure association of the bacteria to DCs by FACS. A representative of three separate experiments from three different donors is shown.
**Figure 3****:** DCs were stimulated with FITC-labelled bacteria at a ratio of 1:50 for 18h in the presence of brefeldin A and then stained for intracellular TNF-α and IL-12. The dot plots show percentage of DC associated with bacteria not producing (lower right quadrant) or producing (upper right quadrant) intracellular cytokines.
**Figure 4****:** TNF-α, IL-12, Il-1β, IL-10 and IL-6 production by DC in response to the *N*. *meningitidis* WT and mutants were measured in culture supernatants by ELISA after 18h of co-culture. Mean and SEM of three separate experiments is shown.
**Figure 5****:** CD40 and CD86 expression after 18h of coculture of the *N. meningitidis* WT and mutants with DCs as indicated by Median Fluorescence Intensity (MFI).
**Figure 6****:** Binding of C-type-lectin-Fc molecules to *N. meninigitidis* WT and mutants in a soluble adhesion assay.
**Figure 7****:** DC association of the *N. meninigitidis* WT and *lgtB* mutant in the presence and absence of 20 microgram anti-DC-SIGN antibody AZN-D1 per ml.
**Figure 8****:** DC-driven Th1/Th2-cell proliferation:
   DC were incubated with *N. meningitidis* wild type H44/76, the *lgtB* mutant, *E. coli* LPS, poly I:C or PGE2 for 48 h, washed and (A) analyzed for maturation markers (mean and SEM of three separate experiments) or (B) co-cultured with highly purified CD45RA⁺CD4⁺ T cells. Quiescent T cells were restimulated with PMA and ionomycin, and intracellular IL-4 (grey) and IFN-γ (black) was analyzed on a single cell basis by flow cytometry. Data from three different donors are shown.

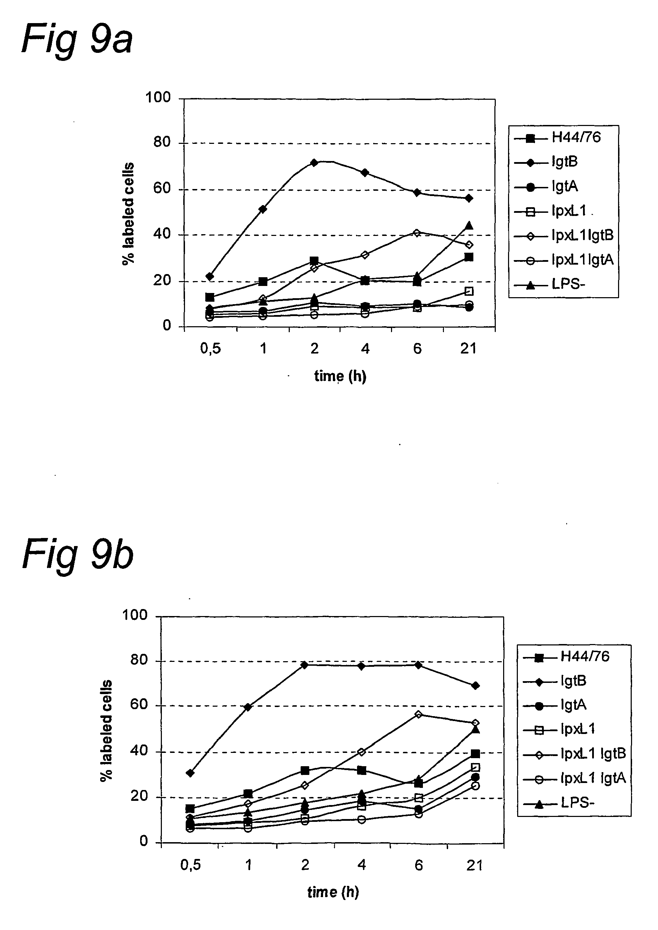

**Figure 9****:** DC association and cytokine induction of live bacteria. Dendritic cells (DC) were cultured with FITC-labelled *N.meningitidis* wild type and LPS mutants at a ratio of 1:100. At the indicated time points samples were taking to measure association of the bacteria to DCs by FACS. Results are shown with cells from two different donors.

### Examples

### 1 Materials and Methods

### 1.1 Bacterial strains

The oligosaccharide core and lipid A mutants were derived from the wild type (WT) group B *N*. *meningitidis* H44/76 and have been described previously:

| Strain or mutant | Reference |
|---|---|
| H44/76 | Holten, 1979, J.Clin.Microbiol. 9: 186-188 |
| *galE* | Jennings et al., 1993, Mol.Microbiol. 10: 361-369 |
| *lgtB* | Jennings et al., 1995, Mol.Microbiol. 18: 729-740 |
| *lpxL1* | van der Ley et al., 2001, Infect-Immun. 69:5981-5990 |

Structure of the oligosaccharide core mutants used in this study are shown in Figure 1. All strains were grown on gonococcal agar (Difco, Basingstoke, UK) supplemented with Vitox (Oxoid Ltd., Basingstoke, UK) in an atmosphere of 6% CO₂ in air at 36 ⁰C. The bacteria were used in stationary phase after culture for 18 hours. Suspensions of bacteria were prepared in RPMI 1640 medium without phenol red (Gibco, Paisley, UK), and their optical density measured at 540nm. Optical density of 1 was calculated to correspond to 10⁹ organisms/ml. Bacteria were fixed in 0.5% paraformaldehyde (PFA) in Phosphate Buffered Saline (PBS) for 15 minutes and washed thoroughly in RPMI medium. FITC labelled bacteria were prepared by incubation with 0.5mg/ml of FITC (Sigma, Poole, UK) for 20 min at 37°C followed by extensive washing.

### 1.2 Cells

DC's were generated from human peripheral blood mononuclear cells (PBMC) as described previously (F. Sallusto and A. Lanzavecchia, 1994, J.Exp.Med. 179: 1109-1118; H. Uronen-Hansson et al., 2004, Immunology 111: 173-178). In brief, monocytes were prepared from PBMC by centrifugation over multistep Percoll gradients. The monocyte fraction was >95% CD14+/CD3-/CD19-. To generate DC's, monocytes were incubated for 7 days in RPMI supplemented with 10% heat inactivated FCS, 2.4mM L-glutamine, 100U/ml Penicillin-streptomycin (all from GIBCO, Paisley UK), 100ng/ml of human recombinant GM-CSF and 50ng/ml of human recombinant IL-4 (Schering-Plough, Welwyn Garden City, Herts UK). Immature DC's prepared in this way were CD14^{low}, CD83^{-ve}, CD86^{low}, CD25^{-ve}. They also expressed HLA DR, HLA DQ, HLA Class I, CD40 and CD1a, and were negative for both CD 19 and CD3.

DC's at a concentration of 5x10⁵/ml were cultured with *N. meningitidis* H44/76 bacteria at DC/bacteria ratio of 1:50 and 1:200 (as indicated in figure legend) in RPMI 1640 supplemented with 10% FCS. When intracellular cytokines were to be measured, the protein transport inhibitor Brefeldin A (Sigma, Poole, UK) was added at 10µg/mL..

The binding specificity of the *lgtB* mutant to DCs was determined by incubating immature DC's with 20 ug/ml anti-DC-SIGN mAb AZN-D1 for 30 min at 37°C, and subsequently with wild-type and mutant bacteria for 45 min at 37°C.

### 1.3 DC binding and internalisation

DC binding and internalisation of bacteria was determined by a combination of flow cytometry and confocal microscopy. For detection by flow cytometry, DC's were incubated with FITC labelled bacteria for periods of time between 30 minutes and 24 hours, fixed in FACSFix (BD), washed and analysed on a FACSCalibur. DC's associated with bacteria were easily identified by fluorescence within the DC gated population. For confocal microscopy, DC's stimulated with FITC labelled *N meningitidis* allowed to adhere for 10 min to an adhesion slide (Bio-Rad Laboratories Ltd., Herts, UK). To stop internalisation, DC's were fixed with 4% PFA for 10 min. DC's were visualized by staining with 5µg/ml of anti MHC Class II monoclonal antibodies (Dako, Glostrup, Denmark). After washing, bound antibody was detected with 5µg/ml of Texas Red -conjugated goat anti-mouse antibody (Molecular probes) for 1h. The slides were washed and mounted in Citifluor (Citifluor Ltd, UK). Confocal images were obtained using a Leica SP2 confocal laser scanning microscope system (Leica, Milton Keynes, UK) fitted with appropriate filter sets. To identify intracellular bacteria, 15-20 optical sections (0.2-0.5µm) spanning the entire DC's were projected and superimposed with Leica confocal imaging software.

### 1.4 Cytokine measurements

To measure intracellular cytokine production, DC's were fixed with 4% PFA in PBS for 15 minutes, washed in PBS containing 0.1% sodium azide and 0.5% BSA (all from Sigma) and permeabilised in 25µl Permeabilisation solution (Caltag). Cells were then incubated with monoclonal antibodies to TNF-α (Becton Dickinson BD, Oxford UK) and IL-12 p40/70 (Pharmingen) or isotype matched controls for 30 minutes at room temperature in the dark. The cells were then washed twice in PBS, fixed in CellFix (Becton Dickinson) and analysed by flow cytometry on a FACScalibur using Cell Quest software (Becton Dickinson). DC's made up a distinct population identified by forward and right angle scattering. At least 95% of cells in this population were DC's as defined by their expression of MHC II, CD1a, CD25, CD80, CD83 and CD86. A minimum of 3000 events within the gates corresponding to DC was collected for analysis. For ELISA assays of soluble cytokines, CytoSets^{™} ELISA kits (Biosource Europe S.A, Nivelles Belgium) for human IL-12, TNF-α and IL-10, IL6 and IL1β were used according to manufacturer's instructions.

### 1.5 Soluble C-type-lectin-Fc Adhesion Assay

C-type-lectin-Fc molecules consist of the extracellular portion of the C-type lectin receptor (DC-SIGN, MGL and Dectin-1B) fused at the COOH terminus to a human IgGl-Fc fragment (T. Geijtenbeek et al., 2003, J.Exp.Med. 197: 7-17). The soluble C-type-lectin-Fc adhesion assay was performed as follows. Whole cells (OD540=0.1) were coated onto ELISA plates (100 ul/well) for 18 hours at RT, followed by blocking with 1% BSA for 30 min at 37°C. Soluble C-type-lectin-Fc supernatant was added for 2h at RT. Unbound C-type-lectin-Fc was washed away and binding was determined by anti-IgG1 ELISA.

### 1.6 DC-driven Th1/Th2 differentation

DC were cultured from monocytes of healthy donors in Iscove's Modified Dulbecco's Medium (Gibco), supplemented with 10% FCS (BioWithaker, Verviers, Belgium), 500 U/ml IL-4 and 800 U/ml GM-CSF (both from Schering-Plough). At day 6 DC maturation was induced with *N. meningitidis* wild type and *lgtB* mutant at a multiplicity of infection of 10. The following positive controls were included in the assay (i) mixed Thl/Th2 response, 10 ng/ml *Escherichia coli* LPS (Sigma-Aldrich, St. Louis, MO) (ii) Th1 differentiation, 20 µg/ml poly I:C (Sigma-Aldrich) and (iii) Th2 differentiation, 10 µg/ml PGE2 10 ng/ml LPS (Sigma-Aldrich). At day 2 DC were analyzed for maturation markers (CD80, CD83, CD86 sand HLA-DR) by flowcytometry. To evaluate T cell differentiation, matured DC were incubated with CD45RA⁺/CD4⁺ T cells (5.10³ T cells/ 20.10³ DC). At day 12-15, quiescent T cells were restimulated with 10 ng/ml PMA ( Sigma-Aldrich) and 1 µg/ml ionomycin (Sigma-Aldrich) for 6 hours. After 1 hour 10 µg /ml Brefeldin A (Sigma-Aldrich) was added to the T cells. Single cell production of IL-4 and IFN-γ was determined by intracellular flowcytometric analysis. Cells were fixed in 2% PFA, permeabilized with 0.5% saponin (Sigma-Aldrich) and stained with anti-human IFN- γ-FITC and anti-human IL-4-PE (BD Pharmingen, San Diego, CA).

### 2. Example: Binding, and internalisation of oligosaccharide core mutant strains of Neisseria meningitidis by DC's

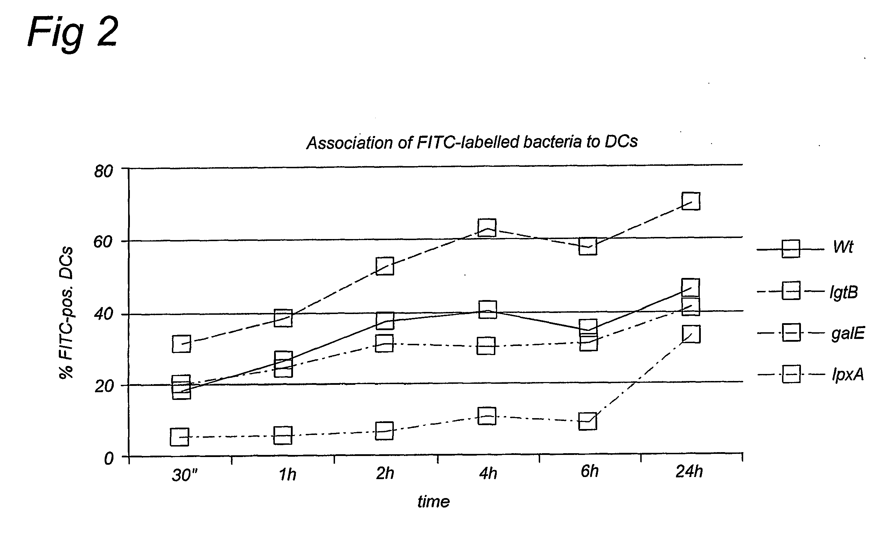

A flow cytometric technique was used to investigate binding and intemalisation of WT *N. meningitidis,* the oligosaccharide mutants and the LPS-deficient mutant by human DC's. Immature DC's were co-cultured with FITC-labelled mutant strains derived from the WT *N. meningitidis* H44/76 at a DC/bacteria ratio of 1:50 (Figure 2) and 1:200 (data not shown) and analysed by FACS for the presence of DC associated FITC-labelled bacteria. Time and dose dependent increase in DC association was observed for both WT and the oligosaccharide mutants. Hardly any DC association was found for the LPS-deficient mutant in the first six hours of the time course. The *lgtB* mutant showed consistently higher DC association than the WT or the *galE* mutants at both concentrations.

To discriminate between bacterial binding and internalisation, we next used confocal microscopy to study DC internalisation of *N. meningitidis* WT and mutants (not shown). FITC bacteria were easily detected as green small particles on slides. MHC Class II staining was used to visualise the DC's. The majority of the DC's had internalised more *lgtB* already after 1h of incubation compared to the WT bacteria or the *galE* mutant. Hardly any intemalisation was seen for the LPS-deficient mutant. In addition to high level of internalisation, surface adherence remained typically high for the *lgtB* mutant bacteria throughout the time course. These results confirm the results by FACS; increased association of bacteria with DC's results in increased internalisation of bacteria.

### 3. Example: Activation and maturation of DC's in response to Neisseria meningitidis mutant strains

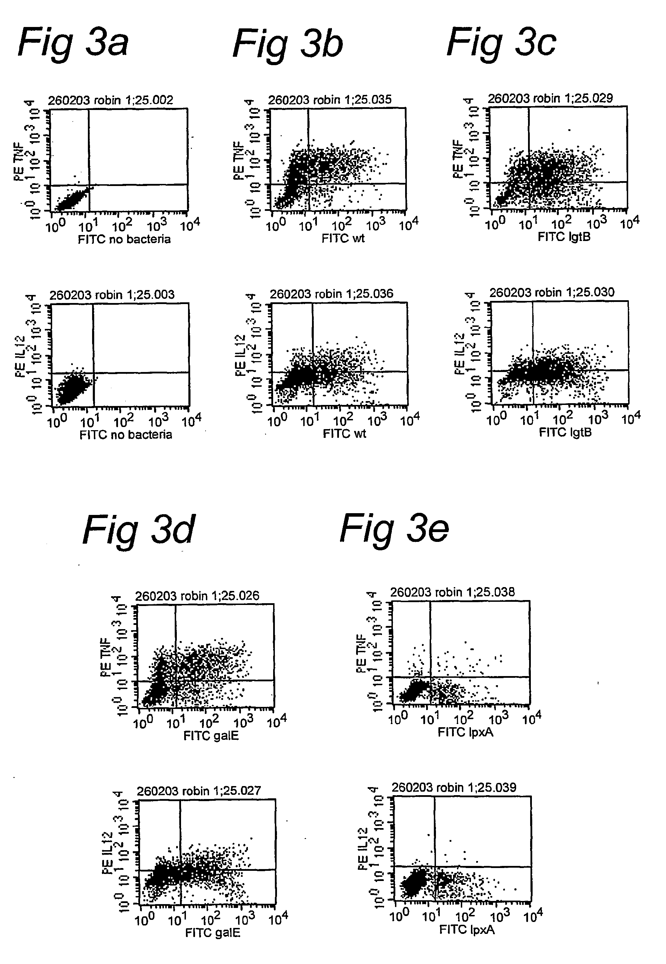

In order to study the relationship between intemalisation of the bacteria and cytokine production, IL12 and TNF-α produced by DC's in response to the WT and the mutants were measured intracellular and simultaneous analysis for intemalisation was performed (Figure 3). DC's were co-cultured with FITC-labelled bacteria at a ratio of 1:50 for 18 hours and then stained for intracellular production of TNF-α and IL12. The quadrants were placed to separate cells that had associated with FITC bacteria from cells that had no FITC bacteria attached. The percentages given are the percentage of DC's producing (upper right quadrant) or not producing (lower right quadrant) intracellular cytokines after internalisation of bacteria. The WT, *lgtB* and *galE* mutant were all potent inducers of TNF-α and IL-12 production by DC's whereas the LPS-deficient mutant was not. Virtually all of the DC's that had internalised *N. meningitidis* WT, the *lgtB* and *galE* mutant were producing high levels of TNF-α. In the case of IL-12, production was found for approximately 50% of the DC's that had internalised the FITC-labelled bacteria.

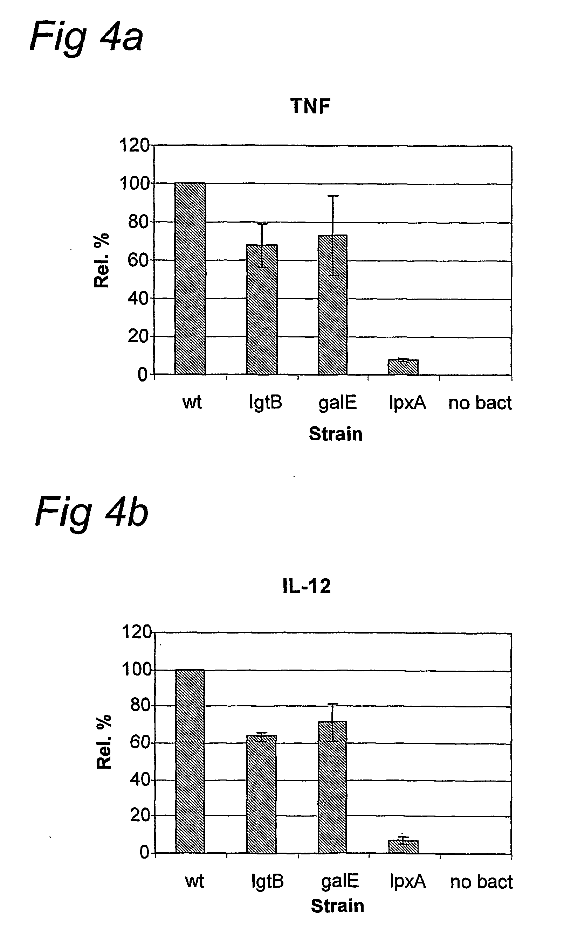

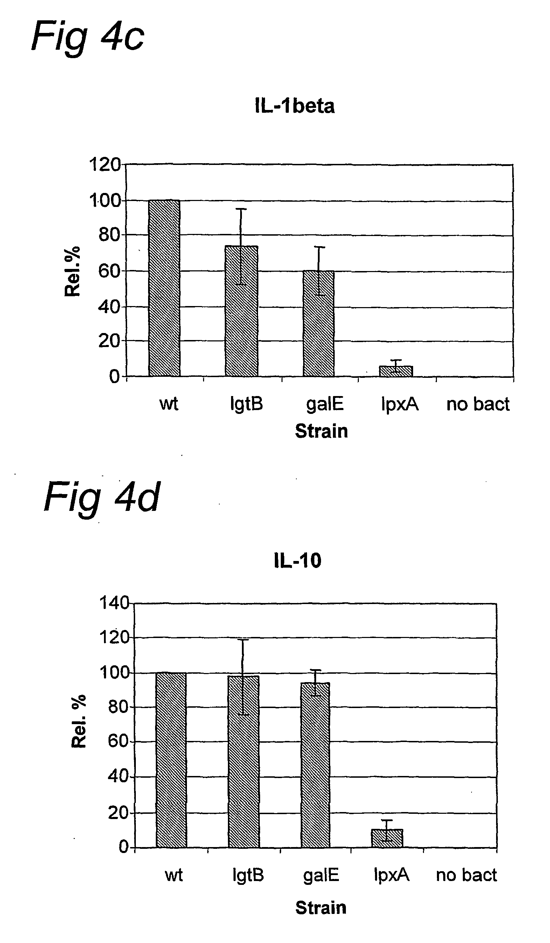

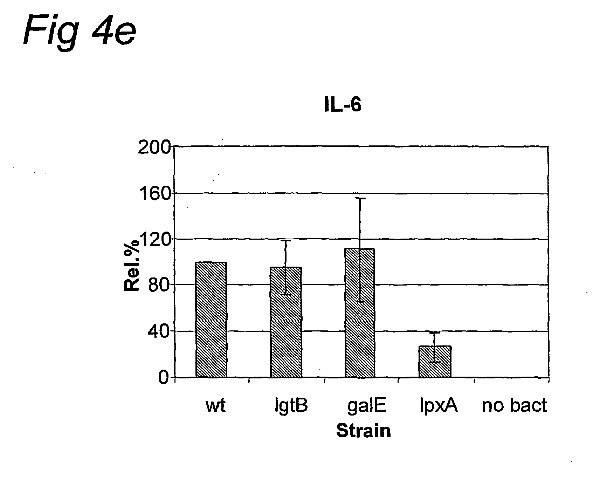

In addition to the intracellular cytokine measurements, we also measured the secreted cytokine levels by DC's in response to *N. meningitidis* (Figure 4). DC's were co-cultured with the mutant and WT bacteria at 1:200 ratio for 18h and the supernatants collected for analysis of secreted IL12, IL10, TNF-α, IL6 and IL-1β. The results are shown as the percentage of cytokine production compared to the WT (WT=100%). Both the *lgtB* and *galE* mutant induced somewhat less IL 12, TNF-α and IL-1β as compared to the WT bacteria. IL-10 and IL-6 production of both *lgtB* and *galE* appears to be similar to the WT strain. Hardly any cytokine production was observed for the LPS-deficient strain.

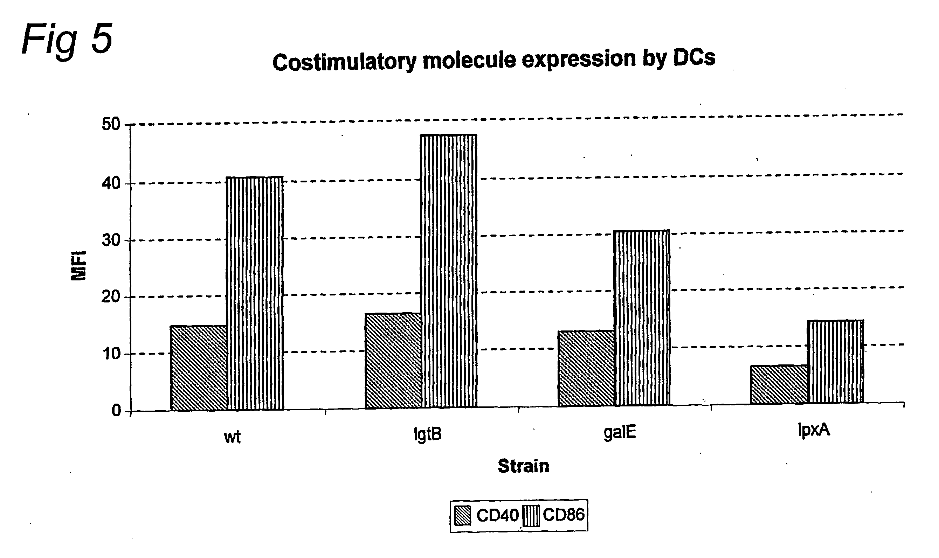

Finally, maturation of DC's stimulated with WT and mutant bacteria was studied by measuring expression of the costimulatory molecules CD40 and CD86 (Figure 5). Expression of CD40 and in particular CD86 was higher when DC's where stimulated with the *lgtB* mutant as compared to DC's stimulated with WT or *galE* mutant bacteria. Hardly any expression of CD40 and CD86 was found when DCs where stimulated with the *lpxA* mutant.

In conclusion, these data demonstrate that binding and internalisation of the *lgtB* mutant by DC's results in activation and maturation of DC's comparable to the level of DC activation and maturation induced with WT bacteria.

### 4. Example: Identification of the receptor mediating_binding and internalisation of the lgtB mutant

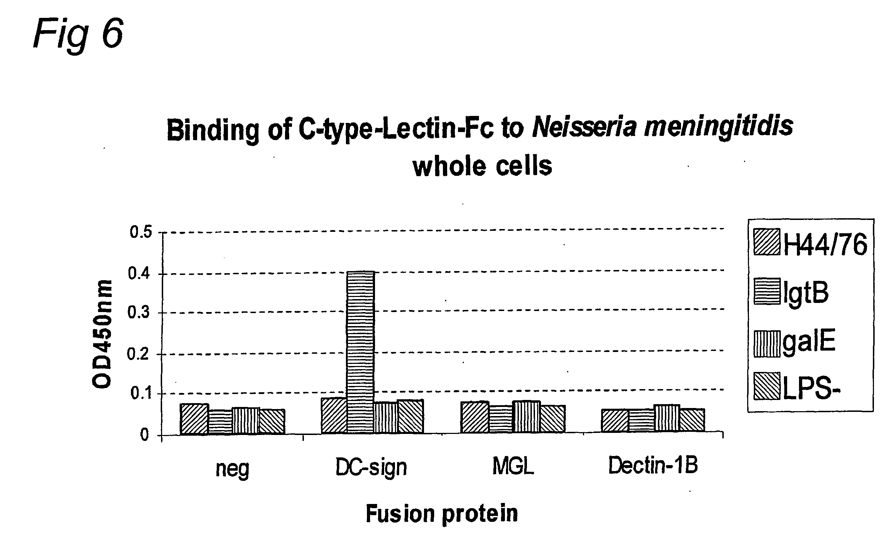

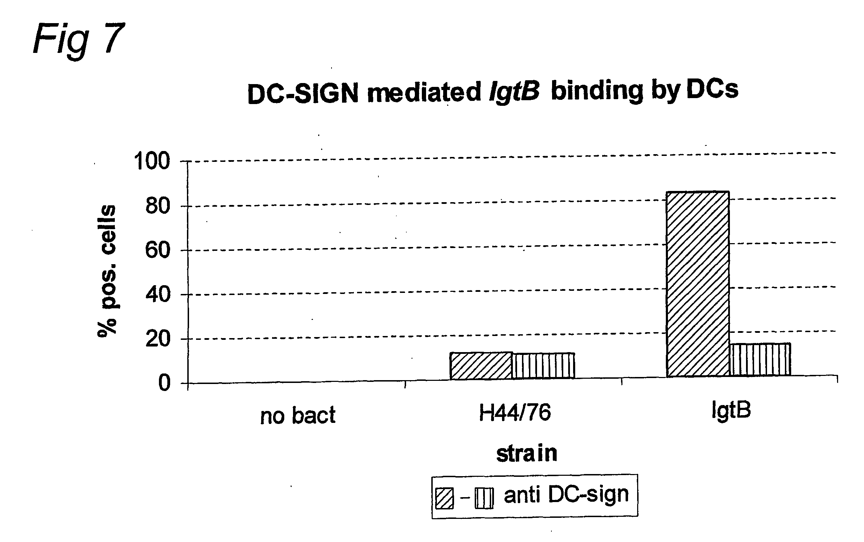

The enhanced binding and uptake by DC's of the *lgtB* mutant prompted us to investigate whether this binding and internalisation is mediated via a specific DC receptor. Therefore we analysed the capability of the WT and the mutants to bind C-type lectin receptors which are capable of recognizing oligosaccharides and are highly expressed on immature DCs. Binding of a panel of C-type-lectin-Fc chimeras to whole cells of WT and mutant bacteria was studied in an soluble adhesion assay (Figure 6). Whereas the WT, the *galE* mutant and the LPS-deficient mutant did not bind any of the C-type-lectin Fc-chimeras, strong binding of DC-SIGN-Fc was found for the *lgtB* mutant. Binding of the *lgtB* mutant to DC-SIGN was also found in HEK293T cells transiently transfected with DC-SIGN or K562 cells stably expressing DC-SIGN (data not shown). Moreover, the specifity of *lgtB* LPS for DC-SIGN was demonstrated by studying DC phagocytosis of the *lgtB* mutant in the presence or absence of the anti-DC-SIGN blocking antibody AZN-D1 (Figure 7). In the presence of AZN-D1, phagocytosis of *lgtB* by DCs was reduced to the level of binding observed for the WT clearly demonstrating that DC-SIGN binds and internalises the *lgtB* mutant.

### 5. Example: Targeting of the lgtB mutant to DC-SIGN initiates a T_{H}1 immune response

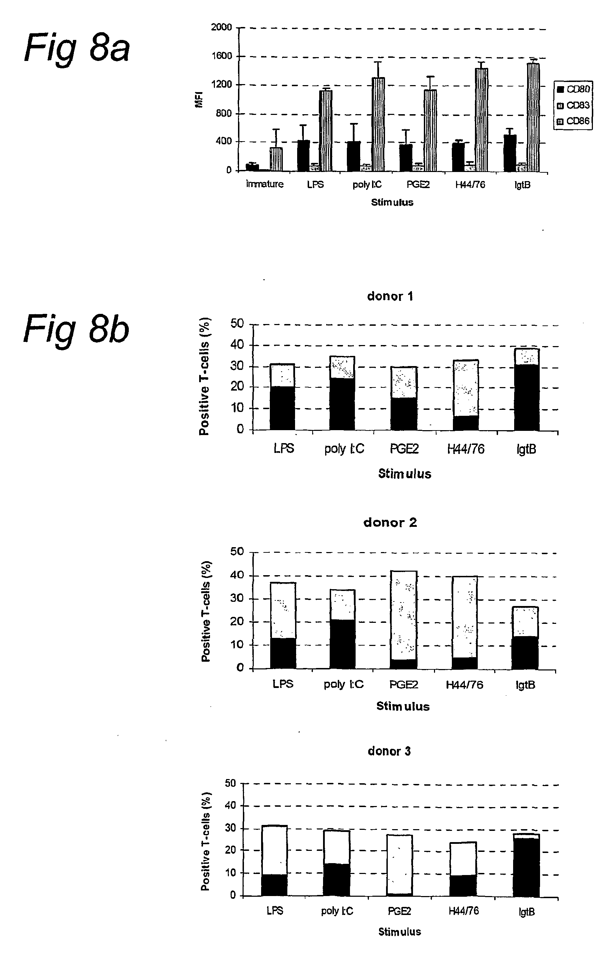

To assess the functional relevance, if any, of the interaction of *lgtB* mutant LPS with DC-SIGN, we studied DC-driven T cell responses after pulsing immature DC with either PFA-fixed wild type or *lgtB* mutant bacteria. No differences, were found in maturation of DCs pulsed with wild type or *lgtB* mutant bacteria as assessed by expression of co-stimulatory molecules and maturation markers (Fig. 8a). However, marked differences were observed in the T_{H}1/T_{H}2-profiles induced with these strains (Fig. 8b). DC pulsed with *N. meningitidis* wild type predominantly generated a T_{H}2 type immune response as the majority of T cells were producing IL-4. DC pulsed with the *lgtB* mutant predominantly evoked IFN-γ-producing T cells, thus shifting the T_{H}1-versus T_{H}2-cell balance towards T_{H}1. This shift in T_{H}1/T_{H}2 balance was observed in 3 independent donors. These data indicate that specific targeting of *lgtB* LPS towards DC-SIGN generates DC signals that drive the immune response into T_{H}1, which is a highly desirable phenomenon for adjuvants. See also P. Moingeon et al. (2001) Vaccine 19:4363-4372.

### 6. Example: Binding and internalisation of oligosaccharide/lipid A double mutant strains of Neisseria meningitidis by DC's

A flow cytometric technique was used to investigate binding and internalisation of WT *N. meningitidis,* the *lgtA* and *lgtB* oligosaccharide mutants and their *lpxL1* double mutants, and the LPS-deficient mutant by human DC's. Immature DC's were co-cultured with FITC-labelled mutant strains derived from the WT *N. meningitidis* H44/76 at a DC/bacteria ratio of 1:100 and analysed by FACS for the presence of DC associated FITC-labelled bacteria (Figure 9). The *lgtb* single mutant showed consistently higher DC association than the WT or the *lgtA* single mutant. In addition, also in the *lpxL1* mutant background the *lgtB* mutation resulted in higher association than either wildtype or *lgtA* oligosaccharide structures, indicating that the increased *lgtB*-mediated binding to DC-SIGN is also possible with LPS containing penta-acylated *lpxL1* lipid A.

## Claims

1. Use of a Neisserial LOS in the manufacture of a medicament for immunisation of a mammal, whereby the Neisserial LOS is represented by the formula (I): 
KDO
α2→4
GlcNAc β1→3 Gal β1→4 Glc β1→4 Hep α1→5 KDO - LA
α1→3
Hep→PEA (3 or 6)
α1→2
GlcNAc ,
 whereby LA is a wild-type Lipid A moiety or a Lipid A moiety with reduced toxicity and whereby the Neisserial LOS is used as an adjuvant.

2. A use according to claims 1, whereby the Neisserial LOS is used in combination with an antigen in the manufacture of a medicament for raising an immune response against the antigen in the mammal.

3. A use according to claim 2, whereby the antigen is from or produced by a bacterium, a virus, a fungus, a parasite, a cancer cell or an allergen.

4. A use according to any one of claims 1 - 3, whereby LA is a Lipid A moiety of which the toxicity is less than 80% of the toxicity of a wild-type Lipid A moiety, as determined in a WEHI assay.

5. A use according to any one of claims 1 - 4, whereby the lipid A moiety is selected from the group consisting of:
(a) a lipid A moiety as obtainable from a *Neisseria* strain with a mutation that reduces the activity or inactivates a *lpxL1* or *lpxL2* gene or a homologue thereof;
(b) a lipid A moiety as obtainable by removal of one or more secondary O-linked esterified fatty acids of a wild-type Lipid A moiety by treatment with an acyloxyhydrolase;
(c) a lipid A moiety as obtainable by hydrazine treatment of a wild-type Lipid A moiety;
(d) a lipid A moiety as obtainable by dephosphorylation with an alkaline phosphatase.

6. A use according to any one of claims 1 - 5, whereby the lipid A moiety is selected from the group consisting of:
(a) a lipid A moiety with a secondary C₁₂-acyl only on the reducing glucosamine;
(b) a lipid A moiety with a secondary C₁₂-aeyl only on the non-reducing glucosamine;
(c) a lipid A moiety lacking both secondary C₁₂-acyl groups;
(d) a lipid A moiety lacking one or more phosphate groups from either the 1 or 4' position; and,
(e) a lipid A moiety with a combination of the deacylations in (a), (b) or (c) and the dephosphorylations in (d).

7. A Neisserial LOS represented by the formula (I): 
KDO
α2→4
GlcNAc β1→3 Gal β1→4 Glc β1→4 Hep α1→5 KDO - LA
α1→3
Hep→PEA (3 or 6)
α1→2
GlcNAc ,
 whereby LA is a wild-type Lipid A moiety or a Lipid A moiety with reduced toxicity, for use as adjuvant in a treatment for immunisation of a mammal.

8. A Neisserial LOS according to claim 7, whereby the Neisserial LOS is used in the treatment in combination with an antigen for raising an immune response against the antigen in the mammal.

9. A Neisserial LOS according to claim 8, whereby the antigen is from or produced by a bacterium, a virus, a fungus, a parasite, a cancer cell or an allergen.

10. A Neisserial LOS according to any one of claims 7 - 9, whereby LA is a Lipid A moiety as defined in any one of claims 4 - 6.

11. A composition comprising a Neisserial LOS represented by the formula (I): 
KDO
α2→4
GlcNAc β1→3 Gal β1→4 Glc β1→4 Hep α1→5 KDO - LA
α1→3
Hep→PEA (3 or 6)
α1→2
GlcNAc ,
 whereby LA is a wild-type Lipid A moiety or a Lipid A moiety with reduced toxicity, whereby the composition further comprises an antigen from or produced by a virus, a fungus, a parasite, a cancer cell, an allergen or a bacterium, whereby the bacterium is selected from the group consisting of *Helicobacter, Haemophilus, Bordetella, Chlamydia, Streptococcus, Vibrio,* a Gram-negative enteric pathogen, *Salmonella, Shigella, Campylobacter, Escherichia,* and bacteria causing anthrax, leprosy, tuberculosis, diphtheria, Lyme disease, syphilis, or typhoid fever, and whereby the composition comprises a pharmaceutically acceptable carrier.

12. A composition according to claim 11, whereby the toxicity of the Lipid A moiety is less than 80% of the toxicity of a wild-type Lipid A moiety, as determined in a WEHI assay.

13. A composition according to claim 11 or 1 2, whereby the lipid A moiety is selected from the group consisting of:
(a) a lipid A moiety as obtainable from a *Neisseria* strain with a mutation that reduces the activity or inactivates a *lpxL1* or *lpxL2* gene or a homologue thereof;
(b) a lipid A moiety as obtainable by removal of one or more secondary O-linked esterified fatty acids of a wild-type Lipid A moiety by treatment with an acyloxyhydrolase;
(c) a lipid A moiety as obtainable by hydrazine treatment of a wild-type Lipid A moiety;
(d) a lipid A moiety as obtainable by dephosphorylation with an alkaline phosphatase.

14. A composition according to any one of claims 11 - 13, whereby the lipid A moiety is:
(a) a lipid A moiety with a secondary C₁₂-acyl only on the reducing glucosamine;
(b) a lipid A moiety with a secondary C₁₂-acyl only on the non-reducing glucosamine;
(c) a lipid A moiety lacking both secondary C₁₂-acyl groups;
(d) a lipid A moiety lacking one or more phosphate groups from either the 1 or 4' position; and,
(e) a lipid A moiety with a combination of the deacylations in (a), (b) or (c) and the dephosphorylations in (d).

## Patentansprüche

1. Verwendung eines Neisseria-LOS in der Herstellung eines Medikaments zur Immunisierung von einem Säuger, wobei das Neisseria-LOS durch die Formel (I) dargestellt ist: 
KDO
α2→4
GlcNAc β1→3 Gal β1→4 Glc β1→4 Hep α1→5 KDO - LA
α1→3
Hep→PEA (3 or 6)
α1→2
GlcNAc ,
 wobei LA ein Wildtyp-Lipid A-Rest oder ein Lipid A-Rest mit verminderter Toxizität ist und wobei das Neisseria-LOS als eine Adjuvanz verwendet wird.

2. Verwendung nach Anspruch 1, wobei das Neisseria-LOS in Kombination mit einem Antigen in der Herstellung eines Medikaments zum Steigern einer Immunantwort gegen das Antigen in dem Säuger verwendet wird.

3. Verwendung nach Anspruch 2, wobei das Antigen aus von einem Bakterium, einem Virus, einem Pilz, einem Parasiten, einer Krebszelle oder einem Allergen stammt oder davon produziert wird.

4. Verwendung nach einem beliebigen der Ansprüche 1-3, wobei LA ein Lipid A-Rest ist, dessen Toxizität weniger als 80 % der Toxizität des Wildtyp-Lipid-A-Rest entspricht, wie bestimmt in einem WEHI-Assay.

5. Verfahren nach einem beliebigen der Ansprüche 1 - 4 , wobei der Lipid-A-Rest ausgewählt ist aus der Gruppe bestehend aus:
(a) einem Lipid-A-Rest, wie erhältlich ist aus einem *Neisseria*-Stamm, mit einer Mutation, die die Aktivität eines *lpxLl-* oder *lpxL2*-Gens oder eines Homologen daraus erniedrigt oder inaktiviert;
(b) einem Lipid-A-Rest, wie erhältlich durch Entfernung einer oder mehrerer sekundärer O-verknüpfter veresteter Fettsäuren von einem Wildtyp-Lipid-A-Rest durch Behandlung mit einer Acyloxyhydrolase;
(c) ein Lipid-A-Rest, wie erhältlich durch Hydrazinbehandlung eines Wildtyp-Lipid-A-Rests;
(d) ein Lipid-A-Rest, wie erhältlich durch Dephosphorylierung mit einer alkalischen Phosphatase.

6. Verwendung nach einem beliebigen der Ansprüche 1 - 5, wobei der Lipid-A-Rest ausgewählt ist aus der Gruppe bestehend aus:
(a) einem Lipid-A-Rest mit einem sekundären C₁₂-Acyl einzig an dem reduzierenden Glukosamin;
(b) einem Lipid-A-Rest mit einem sekundären C₁₂-Acyl einzig an dem nichtreduzierenden Glukosamin;
(c) einem Lipid-A-Rest, dem beide sekundären C₁₂-Acylgruppen fehlen;
(d) einem Lipid-A-Rest, dem eine oder mehrere Phosphatgruppen von einer der 1- oder 4'-Position fehlt/fehlen; und
(e) einem Lipid-A-Rest mit einer Kombination der Deacylierungen in (a), (b) oder (c) und den Dephosphorylierungen in (d).

7. Neisseria-LOS durch die Formel (I) dargestellt: 
KDO
α2→4
GlcNAc β1→3 Gal β1→4 Glc β1→4 Hep α1→5 KDO - LA
α1→3
Hep→PEA (3 or 6)
α1→2
GlcNAc ,
 wobei LA ein Wildtyp-Lipid-A-Rest oder ein Lipid-A-Rest mit reduzierter Toxizität ist, zur Verwendung als Adjuvanz in einer Behandlung zur Immunisierung eines Säugers.

8. Neisseria-LOS nach Anspruch 7, wobei das Neisseria-LOS in der Behandlung in Kombination mit einem Antigen zum Steigern einer Immunantwort gegen das Antigen in Säugern verwendet wird.

9. Neisseria-LOS nach Anspruch 8, wobei das Antigen ist aus oder wird produziert von einem Bakterium, einem Virus, einem Pilz, einem Parasiten, einer Krebszelle oder einem Allergen.

10. Neisseria-LOS nach einem beliebigen der Ansprüche 7 - 9, wobei LA ein Lipid-A-Rest ist wie definiert in einem beliebigen der Ansprüche 4 - 6.

11. Zusammensetzung umfassend ein Neisseria-LOS dargestellt durch die Formel (I): 
KDO
α2→4
GlcNAc β1→3 Gal β1→4 Glc β1→4 Hep α1→5 KDO - LA
α1→3
Hep→PEA (3 or 6)
α1→2
GlcNAc ,
 wobei LA ein Wildtyp-Lipid-A-Rest oder ein Lipid-A-Rest mit reduzierter Toxizität ist, wobei die Zusammensetzung ferner ein Antigen aus oder produziert von einem Virus, einem Pilz, einem Parasiten, einer Krebszelle, einem Allergen oder einem Bakterium umfasst, wobei das Bakterium ausgewählt ist aus einer Gruppe bestehend aus *Helicobacter, Haemophilus, Bordetella, Chlamydia, Streptococcus, Vibrio, einem gram-negativen Enteropathogen, Salmonella, Shigella, Campylobacter, Escherichia,* und Bakterien, welche Milzbrand, Lepra, Tuberkulose, Diphtherie, Borreliose, Syphilis, oder Typhus hervorrufen, und wobei die Zusammensetzung einen pharmazeutisch verträglichen Träger umfasst.

12. Zusammensetzung nach Anspruch 11, wobei die Toxizität des Lipid-A-Rests weniger als 80 % der Toxizität des Wildtyp-Lipid-A-Rests beträgt, wie bestimmt in einen WEHI-Assay.

13. Zusammensetzung nach Anspruch 11 oder 12, wobei der Lipid-A-Rest ausgewählt ist aus der Gruppe bestehend aus:
(a) einem Lipid-A-Rest, wie erhältlich aus einem *Neisseria*-Stamm mit einer Mutation, die die Aktivität eines *lpxL1-* oder *lpxL2*-Gens oder einem Homologen daraus erniedrigt oder inaktiviert;
(b) einem Lipid-A-Rest, wie erhältlich durch Entfernung von einem oder mehrerer O-verknüpfter veresteter Fettsäuren oder einem Wildtyp-Lipid-A-Rest durch Behandlung mit einer Acyloxyhydrolase;
(c) einem Lipid-A-Rest, wie erhältlich durch Hydrazinbehandlung von einem Wildtyp-Lipid-A-Rests;
(d) einem Lipid-A-Rest, wie erhältlich durch Dephosphorylierung mit einer alkalischen Phosphatase.

14. Zusammensetzung nach einem beliebigen der Ansprüche 11 - 13, wobei der Lipid-A-Rest ist:
(a) ein Lipid-A-Rest mit einem sekundären C₁₂-Acyl einzig an dem reduzierenden Glukosamin;
(b) ein Lipid-A-Rest mit einem sekundären C₁₂-Acyl einzig an dem nichtreduzierenden Glukosamin;
(c) einem Lipid-A-Rest, dem beide sekundäre C₁₂-Acylgruppen fehlen;
(d) einem Lipid-A-Rest, dem eine oder mehrere Phosphatgruppen an der 1- oder 4'-Position fehlt/fehlen; und
(e) ein Lipid-A-Rest mit einer Kombination der Deacylierungen in (a), (b) oder (c) und den Dephosphorylierungen in (d).

## Revendications

1. Utilisation d'un LOS de *Neisseria* dans la fabrication d'un médicament destiné à l'immunisation d'un mammifère, où le LOS de *Neisseria* est représenté par la formule (1) : 
KDO
α2→4
GlcNAc β1→3 Gal β1→4 Glc β1→4 Hep α1→5 KDO - LA
α1→3
Hep→PEA (3 or 6)
α1→2
GlcNAc ,
 dans laquelle LA est un fragment de lipide A de type sauvage ou un fragment de lipide A avec une toxicité réduite et où le LOS de *Neisseria* est utilisé en tant qu'adjuvant.

2. Utilisation selon la revendication 1, où le LOS de *Neisseria* est utilisé en combinaison avec un antigène dans la fabrication d'un médicament destiné à déclencher une réponse immunitaire contre l'antigène chez le mammifère.

3. Utilisation selon la revendication 2, où l'antigène provient de ou est produit par une bactérie, un virus, un champignon, un parasite, une cellule cancéreuse ou un allergène.

4. Utilisation selon l'une quelconque des revendications 1 à 3, où LA est un fragment de lipide A dont la toxicité est inférieure à 80 % de la toxicité d'un fragment de lipide A de type sauvage, déterminée dans un test sur cellule WEHI.

5. Utilisation selon l'une quelconque des revendications 1 à 4, où le fragment de lipide A est choisi dans le groupe constitué par :
(a) un fragment de lipide A tel que pouvant être obtenu à partir d'une souche de *Neisseria* avec une mutation qui réduit l'activité ou inactive un gène *lpxL1* ou *lpxL2* ou un homologue de ceux-ci ;
(b) un fragment de lipide A tel que pouvant être obtenu par l'élimination d'un ou plusieurs acides gras estérifiés O-liés d'un fragment de lipide A de type sauvage par traitement avec une acyloxyhydrolase ;
(c) un fragment de lipide A tel que pouvant être obtenu par un traitement par hydrazine d'un fragment de lipide A de type sauvage ;
(d) un fragment de lipide A tel que pouvant être obtenu par déphosphorylation avec une phosphatase alcaline.

6. Utilisation selon l'une quelconque des revendications 1 à 5, où le fragment de lipide A est choisi dans le groupe constitué par :
(a) un fragment de lipide A avec un groupe acyle en C₁₂ secondaire seulement sur la glucosamine réductrice ;
(b) un fragment de lipide A avec un groupe acyle en C₁₂ secondaire seulement sur la glucosamine non réductrice ;
(c) un fragment de lipide A manquant des deux groupes acyle en C₁₂ secondaires ;
(d) un fragment de lipide A manquant d'un ou plusieurs groupes phosphate à partir de la position soit 1 soit 4' ; et
(e) un fragment de lipide A avec une combinaison des désacylations en (a), (b) ou (c) et des déphosphorylations en (d).

7. LOS de *Neisseria* représenté par la formule (1) : 
KDO
α2→4
GlcNAc β1→3 Gal β1→4 Glc β1→4 Hep α1→5 KDO - LA
α1→3
Hep→PEA (3 or 6)
α1→2
GlcNAc ,
 dans laquelle LA est un fragment de lipide A de type sauvage ou un fragment de lipide A avec une toxicité réduite, utilisable en tant qu'adjuvant dans un traitement d'immunisation d'un mammifère.

8. LOS de *Neisseria* selon la revendication 7, où le LOS de *Neisseria* est utilisé dans le traitement en combinaison avec un antigène pour déclencher une réponse immunitaire contre l'antigène chez le mammifère.

9. LOS de *Neisseria* selon la revendication 8, où l'antigène provient de ou est produit par une bactérie, un virus, un champignon, un parasite, une cellule cancéreuse ou un allergène.

10. LOS de *Neisseria* selon l'une quelconque des revendications 7 à 9, où LA est un fragment de lipide A tel que défini dans l'une quelconque des revendications 4 à 6.

11. Composition comprenant un LOS de *Neisseria* représenté par la formule (1) : 
KDO
α2→4
GlcNAc β1→3 Gal β1→4 Glc β1→4 Hep α1→5 KDO - LA
α1→3
Hep→PEA (3 or 6)
α1→2
GlcNAc ,
 dans laquelle LA est un fragment de lipide A de type sauvage ou un fragment de lipide A avec une toxicité réduite, où la composition comprend en outre un antigène de ou produit par un virus, un champignon, un parasite, une cellule cancéreuse, un allergène ou une bactérie, où la bactérie est choisie dans le groupe constitué par *Helicobacter, Haemophilus, Bordetella, Chlamydia, Streptococcus, Vibrio,* un agent pathogène entérique Gram négatif, *Salmonella, Shigella, Campylobacter, Escherichia* et des bactéries provoquant l'anthrax, la lèpre, la tuberculose, la diphtérie, la maladie de Lyme, la syphilis ou la fièvre typhoïde, et où la composition comprend un support pharmaceutiquement acceptable.

12. Composition selon la revendication 11, dans laquelle la toxicité du fragment de lipide A est inférieure à 80 % de la toxicité d'un fragment de lipide A de type sauvage, déterminée dans un test sur cellule WEHI.

13. Composition selon la revendication 11 ou 12, dans laquelle le fragment de lipide A est choisi dans le groupe constitué par :
(a) un fragment de lipide A tel que pouvant être obtenu à partir d'une souche de *Neisseria* avec une mutation qui réduit l'activité ou inactive un gène *lpxL1* ou *lpxL2* ou un homologue de ceux-ci ;
(b) un fragment de lipide A tel que pouvant être obtenu par l'élimination d'un ou plusieurs acides gras estérifiés O-liés d'un fragment de lipide A de type sauvage par traitement avec une acyloxyhydrolase ;
(c) un fragment de lipide A tel que pouvant être obtenu par un traitement par hydrazine d'un fragment de lipide A de type sauvage;
(d) un fragment de lipide A tel que pouvant être obtenu par déphosphorylation avec une phosphatase alcaline.

14. Composition selon l'une quelconque des revendications 11 à 13, dans laquelle le fragment de lipide A est :
(a) un fragment de lipide A avec un groupe acyle en C₁₂ secondaire seulement sur la glucosamine réductrice ;
(b) un fragment de lipide A avec un groupe acyle en C₁₂ secondaire seulement sur la glucosamine non réductrice ;
(c) un fragment de lipide A manquant des deux groupes acyle en C₁₂ secondaires ;
(d) un fragment de lipide A manquant d'un ou plusieurs groupes phosphate à partir de la position soit 1 soit 4' ; et
(e) un fragment de lipide A avec une combinaison des désacylations en (a), (b) ou (c) et des déphosphorylations en (d).
